# FASCICULE DE BREVET EUROPEEN

(11) **EP 1 363 603 B1**
(45) Date de publication et mention de la délivrance du brevet: **01.06.2005**
(21) Numéro de dépôt: 02706888.1
(22) Date de dépôt: 22.02.2002
(51) Int. Cl.: A61K 9/16, A61K 7/16

(54) **PROCEDE POUR RENDRE HYDROSOLUBLE UN ANTISEPTIQUE PEU SOLUBLE DANS L'EAU**
VERFAHREN ZUM WASSERLÖSLICHMACHEN EINES SCHWERWASSERLÖSLICHEN ANTISEPTIKUMS
METHOD FOR MAKING WATER SOLUBLE A POORLY WATER-SOLUBLE ANTISEPTIC

(30) Priorité: 01.03.2001 FR 0102794
(43) Date de publication de la demande: 26.11.2003
(73) Titulaire: CLL Pharma, 06200 Nice (FR)
(72) Inventeur: TOSELLI, Dominique, F-06000 NICE (FR); ZAKARIAN, Noel, F-13009 MARSEILLE (FR)
(74) Mandataire: Goulard, Sophie
(86) Numéro de dépôt international: PCT/FR2002/000663
(87) Numéro de publication internationale: WO 2002/069931

(56) Documents cités:
- WO-A-00/23040
- FR-A- 2 613 223
- FR-A- 2 649 611

## Description

L'invention se rapporte à un procédé de préparation de granulés hydrosolubles renfermant au moins un antiseptique naturellement peu ou pas soluble dans l'eau, ainsi qu'aux utilisations de ce procédé, et notamment pour la préparation de compositions pharmaceutiques se présentant sous forme sèche et permettant de réaliser extemporanément des solutions pour bains de bouche.

Il est usuel que les médecins et les dentistes prescrivent, en cas d'affections de la cavité buccale (aphtes, gingivites, parodontites superficielles, ...) ou de l'oropharynx, ainsi que dans les suites opératoires en stomatologie et parodontologie, des bains de bouche ou gargarismes qui doivent être effectués plusieurs fois par jour.

Les solutions pour bains de bouche commercialement disponibles à ce jour (HEXTRIL®, CORSODYL®, COLLUNOVAR®, ELUDRIL®, GIVALEX®, ALODONT®, PREXIDINE®, ...) sont des solutions hydro-éthanoliques, qui sont conditionnées dans des flacons en verre ou en plastique, d'une contenance allant de 125 à 500 ml, et qui sont peu pratiques à utiliser dès lors que l'on est amené à se déplacer, compte tenu de leur encombrement et des risques de casse et/ou de souillure en cas de mauvaise fermeture du flacon. Il en résulte le plus souvent une mauvaise observance du traitement par les patients qui évitent de se déplacer avec leur flacon de solutions pour bains de bouche.

Il serait donc souhaitable, pour permettre aux patients de mieux suivre leur traitement, de leur proposer des compositions se présentant sous forme sèche, de préférence conditionnées en doses unitaires, facilement transportables, et aptes à donner, après dispersion dans un volume d'eau approprié, des solutions pour bains de bouche.

Or, il se trouve que la plupart des principes actifs à visée antiseptique susceptibles d'être utilisés dans des solutions pour bains de bouche comme l'hexétidine et ses dérivés, la chlorhexidine et bon nombre de ses sels, le chlorobutanol hémihydraté et l'eugénol, sont solubles dans les solvants organiques mais ne le sont pas ou que très peu dans l'eau. Aussi, leur dispersion dans l'eau conduit à la formation d'une suspension trouble, d'un aspect peu attrayant, et dans laquelle le principe actif n'est pas uniformément réparti, ce qui peut se traduire par une diminution de l'efficacité thérapeutique des bains de bouche.

Pour obtenir une solubilisation satisfaisante de ces substances, il convient d'utiliser un solvant organique, l'éthanol étant le plus adapté à une utilisation pharmaceutique. Toutefois, pour des raisons évidentes de santé publique et aussi de commodité, il n'est pas envisageable de proposer des compositions pour bains de bouche devant être reconstituées, partiellement ou totalement, dans de l'éthanol.

On connaît de longue date des comprimés effervescents destinés à permettre la préparation extemporanée de solutions pour bains de bouche. De tels comprimés sont, par exemple, décrits dans les Brevets Américains n° 3,772,431, 3,888,976 et 4,919,918. Il s'agit dans tous les cas de produits grand public, ayant davantage vocation à permettre un nettoyage de la cavité buccale et des dents, par un effet effervescent, voire moussant, qu'une désinfection. De ce fait, dans ces Brevets, la présence d'un antiseptique comme le chlorure de cétyldiméthyl benzylammonium ou la chlorhexidine, n'est envisagée que de manière très accessoire. Il est à noter que le Brevet Américain n° 4,919,918 propose, pour assurer une solubilisation dans l'eau des substances peu ou pas solubles susceptibles d'être présentes dans les comprimés, d'ajouter un agent tensioactif du type laurylsulfate, n-laurylsarcosinate ou alkylsulfoacétate de sodium, qui contribue à l'effet moussant recherché. Un tel effet moussant n'est, toutefois, pas souhaitable pour des bains de bouche à visée thérapeutique.

Il a par ailleurs déjà été proposé des procédés permettant de rendre hydrosolubles des principes actifs naturellement peu ou pas solubles dans l'eau.

Ainsi, la demande de brevet FR-A-2 649 611 propose un procédé de préparation d'une composition thérapeutique à base d'acide acétylsalicylique consistant à dissoudre ce principe actif dans un solvant puis à imprégner de la solution ainsi formée un support alimentaire glucidique puis à provoquer l'évaporation du solvant pour conduire à la formations de microparticules ou de microcristaux du principe actif, le développement des cristaux étant limité par la matrice du support.

Par ailleurs, la demande de brevet FR-A-2 613 223 propose un procédé de préparation d'une forme galénique solide se présentant sous la forme de grains hydrosoluble renfermant du Ginkgo biloba. Selon ce procédé, ce principe actif et solubilisé dans un solvant en présence d'un agent tensioactif, puis cette solution est ensuite mélangée avec un support hydrosoluble qui est ensuite soumis à une granulation. Selon ce document, le support hydrosoluble peut indifféremment être choisi parmi le saccharose, le lactose, le mannitol et les maltodextrines.

Cependant, si ces procédés permettent d'améliorer la solubilité de ces principes actifs particuliers que sont l'acide acétylsalicylique et le Ginkgo biloba quelle que soit la nature du support hydrosoluble utilisé, ils ne donnent pas entièrement satisfaction lorsqu'ils sont appliqués pour solubiliser des principes actifs-antiseptiques.

Les Inventeurs se sont, donc, fixé pour but de fournir un procédé qui permette de rendre hydrosoluble un antiseptique naturellement peu ou pas soluble dans l'eau, de manière à être en mesure de préparer des compositions pharmaceutiques se présentant sous forme sèche et qui, bien que renfermant un ou plusieurs antiseptiques peu ou pas hydrosolubles, soient capables de former des solutions homogènes et stables (sans aucun phénomène de déphasage) après dispersion dans l'eau.

Les Inventeurs se sont, de plus, fixé pour but de fournir un procédé qui, tout en présentant ces avantages, soit simple à mettre en oeuvre et ne nécessite pas d'autres appareillages que ceux dont sont classiquement équipés les Laboratoires pharmaceutiques.

L'invention a, donc, pour objet un procédé de préparation de granulés hydrosolubles renfermant au moins un antiseptique, comprenant :
a) une étape de granulation par voie humide ou d'imprégnation d'un support hydrosoluble pharmaceutiquement acceptable sous forme de granulés au moyen d'un solvant renfermant au moins un principe actif, et
b) une étape de séchage des granulés,
caractérisé par le fait que le support hydrosoluble sous forme de granulés est du mannitol et que le principe actif est un antiseptique.

Dans le cadre de leurs travaux, les Inventeurs ont en effet constaté que, de manière surprenante, lorsqu'on soumet du mannitol à une granulation par voie humide ou à une imprégnation, au moyen d'un solvant contenant un antiseptique naturellement peu ou pas soluble dans l'eau, cet antiseptique se lie au mannitol sans être dénaturé, et reste lié à ce dernier lorsque les granulés sont ultérieurement dispersés dans l'eau. On obtient alors une solution au sein de laquelle l'antiseptique est parfaitement dissous et propre à exercer ses effets thérapeutiques, comme si sa .solubilisation était rendue possible par la propriété très hydrophile du mannitol auquel il est lié.

Les Inventeurs n'ont pas encore identifié la nature de la liaison qui s'établit entre le principe actif antiseptique et le mannitol au cours de la granulation. Par contre, l'existence de cette liaison a pu clairement être mise en évidence par des tests montrant que, si l'on soumet une solution obtenue par dispersion des granulés dans l'eau, à des filtrations et des extractions, on retrouve constamment le principe actif antiseptique dans la fraction où se trouve le mannitol et ce sans qu'aucune trace d'antiseptique non lié au mannitol ne soit détectée.

Par contre, lorsqu'on soumet du xylitol ou du sorbitol à une granulation par voie humide ou à une imprégnation, au moyen d'un solvant contenant le même antiseptique, puis à la dispersion des granulés obtenus dans l'eau, on constate que l'antiseptique ne reste pas complètement lié à ce dernier lorsque les granulés sont ultérieurement dispersés dans l'eau.

Selon une disposition avantageuse de ce procédé, le solvant utilisé pour la granulation ou l'imprégnation est l'eau, un alcool ou un mélange eau-alcool. Les Inventeurs ont, en effet, constaté qu'il n'est pas nécessaire, pour que l'antiseptique se lie convenablement au mannitol, qu'il soit dissous dans le solvant, en sorte que ce dernier peut être aussi bien l'eau dans laquelle l'antiseptique se trouve en suspension qu'un alcool ou un mélange hydro-alcoolique dans lequel il se trouve en solution.

Lorsque le solvant est un alcool ou un mélange eau-alcool, on utilise de préférence l'éthanol. Toutefois, d'autres alcools comme l'isopropanol, sont également susceptibles d'être utilisés pour la mise en oeuvre du procédé conforme à l'invention.

Selon une autre disposition avantageuse du procédé conforme à l'invention, l'antiseptique est choisi parmi les ammonium quaternaires, l'hexétidine, les bisbiguanides, la chlorhexidine et ses sels tels que le chlorhydrate ou l'acétate de chlorhexidine, le chlorobutanol hémihydraté, l'eugénol, l'eucalyptol, la tyrothricine et les huiles essentielles comme l'huile essentielle de menthe poivrée.

Conformément à l'invention, cet antiseptique peut être associé à un
ou plusieurs autres principes actifs, antiseptique ou non, en fonction de l'effet thérapeutique recherché, lesquels peuvent soit présenter également une solubilité dans l'eau faible ou nulle, soit être, eux, parfaitement solubles dans l'eau, comme c'est notamment le cas du digluconate de chlorhexidine, du salicylate de choline et de certains ammonium quaternaires connus pour présenter une bonne hydrosolubilité (chlorure de cétyl pyridinium par exemple) que l'on associe couramment au chlorobutanol hémihydraté et à l'hexétidine.

Lorsque les principes actifs que l'on souhaite associer ont des solubilités très différentes, il est préférable de les dissoudre dans deux solvants différents (par exemple, un alcool pour le principe actif le moins soluble, l'eau ou un mélange eau-alcool pour le principe actif le plus soluble) et d'effectuer la granulation du mannitol en mouillant ce dernier d'abord par le solvant contenant le principe actif le moins soluble, puis par le solvant contenant le principe actif le plus soluble.

Par ailleurs, le solvant utilisé pour la granulation ou l'imprégnation du mannitol peut comprendre, de plus, un ou plusieurs excipients comme un colorant (amarante, rouge cochenille, bleu patenté V, ...) choisi pour sa compatibilité avec l'antiseptique utilisé et le ou les principes actifs additionnels éventuels et destiné à conférer une couleur agréable à la solution résultant de la dispersion des granulés dans l'eau, ou tout autre excipient que l'on souhaite associer à l'antiseptique et qui présente, lui aussi, une solubilité dans l'eau faible ou nulle.

Selon encore une autre disposition avantageuse du procédé conforme à l'invention, la granulation ou l'imprégnation est réalisée avec une quantité de solvant correspondant à 10 à 80% et, de préférence, à 15 à 75% en poids du poids du mannitol, tandis que ce solvant présente une teneur pondérale en antiseptique comprise entre 10 et 80% et, de préférence, entre 15 et 70%. Ainsi, à l'issue du séchage des granulés, ces derniers présentent avantageusement un rapport pondéral entre le mannitol et l'antiseptique compris entre 0.5 et 50 et, de préférence, entre 1 et 30.

Conformément à l'invention, la granulation du mannitol et le séchage des granulés sont, de préférence, réalisés dans un lit d'air fluidisé à une température pouvant varier entre 20 et 60°C selon le degré de sensibilité à la chaleur du ou des antiseptiques et du ou des principes actifs additionnels éventuellement utilisés. Toutefois, il est également possible d'effectuer la granulation du mannitol dans un mélangeur planétaire et de n'utiliser un lit d'air fluidisé que pour le séchage des granulés. Ce séchage peut également être réalisé à l'aide d'une étuve.

Selon encore une autre disposition avantageuse du procédé conforme à l'invention, celui-ci comprend, postérieurement au séchage des granulés, une opération de calibrage de ces granulés, par exemple par tamisage sur une grille, ce calibrage étant, de préférence, conduit de manière à éliminer tous les granulés présentant une taille supérieure à 5 mm.

Le procédé qui vient d'être décrit présente de nombreux avantages. En effet, en permettant de rendre soluble dans l'eau des antiseptiques qui naturellement ne le sont pas ou peu, il offre la possibilité de réaliser, à partir d'antiseptiques à hydrosolubilité faible ou nulle, des compositions se présentant sous forme sèche et .aptes à former des solutions homogènes et stables après dispersion dans l'eau. Par ailleurs, ce procédé est simple à mettre en oeuvre et n'utilise que des matières premières (mannitol, eau, alcool) aisément disponibles et d'un coût compatible avec une exploitation à une échelle industrielle. Enfin, il ne nécessite pas d'équipement spécifique et peut être mis en oeuvre au moyen des appareillages dont sont classiquement équipés les Laboratoires pharmaceutiques, la granulation par voie humide et l'imprégnation étant en effet couramment utilisées pour la fabrication de médicaments proposés en gélules et en comprimés.

L'invention a, également, pour objet l'utilisation d'un procédé tel que précédemment défini pour la préparation d'une composition pharmaceutique se présentant sous forme sèche et apte à donner extemporanément une solution pour bains de bouche, après dispersion dans l'eau.

L'invention a donc également pour objet une composition pharmaceutique se présentant sous une forme sèche et permettant de réaliser de manière extemporanée une solution pour bains de bouche, par dispersion dans l'eau, qui est caractérisée en ce qu'elle comprend des granulés formés de mannitol lié à au moins un antiseptique.

Selon une disposition préférée de cette composition, l'antiseptique est choisi parmi les ammonium quaternaires, l'hexétidine, les bisbiguanides, la chlorhexidine et ses sels tels que le chlorhydrate ou l'acétate de chlorhexidine, le chlorobutanol hémihydraté, l'eugénol, l'eucalyptol, la tyrothricine et les huiles essentielles comme celle de menthe poivrée.

De manière particulièrement préférée, l'antiseptique est choisi parmi l'hexétidine et le chlorobutanol hémihydraté, éventuellement en association avec du digluconate de chlorhexidine.

Conformément à l'invention, la composition peut être constituée uniquement par des granulés tels qu'obtenus par le procédé précédemment décrit.

Toutefois, bien que le mannitol soit lui-même un édulcorant et contribue à donner à la composition à la fois une douceur et une fraîcheur en bouche, on préfèrera le plus souvent mélanger les granulés à un ou plusieurs édulcorants tels que le cyclamate de sodium, le saccharinate de sodium, l'aspartam ou le glycérinate d'ammonium, et/ou un ou plusieurs arômes (menthe, anis, citron, framboise, ...) pour la rendre encore plus agréable, tant au niveau du goût que de son parfum, notamment dans le cas où l'antiseptique utilisé présente des qualités organoleptiques peu avantageuses.

Par ailleurs, la composition peut se présenter sous la forme d'une poudre résultant du mélange des granulés avec le ou les excipients, ou sous la forme de comprimés. Dans ce dernier cas, la composition comprend encore un ou plusieurs excipients choisis parmi ceux classiquement utilisés pour la compression de poudres ou de granulés comme les agents d'écoulement, les agents de remplissage et les agents lubrifiants.

Selon une autre disposition préférée de la composition conforme à l'Invention, cette dernière est conditionnée en doses unitaires correspondant chacune à un bain de bouche. De manière particulièrement préférée, chaque dose unitaire est conditionnée dans un emballage étanche à l'air et à l'eau, comme par exemple un sachet formé d'une feuille d'aluminium doublée d'un film de polyéthylène, et dont les dimensions sont choisies de manière à pouvoir contenir, une fois ouvert, un volume d'eau suffisant pour effectuer un bain de bouche, ce qui permet d'éviter ainsi l'emploi d'un verre que l'on n'a pas toujours à portée de main, notamment en cas de déplacement.

La composition conforme à l'invention présente de nombreux avantages. En effet, elle se disperse facilement dans l'eau pour former une solution limpide, homogène, stable et d'un goût très agréable, même lorsqu'elle contient un antiseptique aux propriétés organoleptiques peu avantageuses. Etant sous une forme sèche, elle supprime les risques de dégradation des antiseptiques ayant tendance à être instables en solution comme c'est principalement le cas de la chlorhexidine dont la stabilité dépend étroitement du degré alcoolique et du pH. Présentée en dosages unitaires, elle garantit l'utilisation d'une dose efficace d'antiseptique lors de chaque bain de bouche. Enfin, de par sa présentation et sa facilité d'emploi, elle offre la possibilité d'effectuer un bain de bouche en toute circonstance sans avoir à se déplacer avec un flacon susceptible de se casser ou de s'ouvrir de façon intempestive.

L'invention a, en outre, pour objet une solution pour bains de bouche, caractérisée en ce qu'elle est obtenue en dispersant dans l'eau une composition pharmaceutique telle que précédemment définie.

L'invention sera mieux comprise à l'aide du complément de description qui suit, qui se réfère à des exemples de préparation de compositions sèches destinées à être utilisées pour la réalisation extemporanée de solutions pour bains de bouche et de démonstration de l'aptitude de ces compositions à former des solutions homogènes et stables après dispersion dans l'eau.

Il va de soi, toutefois, que ce complément de description n'est donné qu'à titre d'illustrations de l'invention et n'en constitue en aucune manière une limitation.

### EXEMPLE 1 : Composition comprenant l'hexétidine en tant que principe actif

On réalise 100 000 sachets renfermant chacun 540,54 mg d'une composition se présentant sous la forme de granulés et ayant la formulation suivante :
Par sachet
- Hexétidine 5,000 mg
- Colorant amarante E123 0,540 mg
- Mannitol granulé 495,000 mg
- Aspartam micronisé 8,000 mg
- Arôme menthe 32,000 mg par un procédé comprenant :

- la dissolution de 525 g d'hexétidine et de 56.7 g d'amarante E123 dans 8,84 litres d'éthanol ;
- la mise en fluidisation pendant 15 minutes de 51,975 kg de mannitol granulé dans un lit d'air fluidisé présentant une température de 40°C ;
- l'aspersion du mannitol granulé par la solution éthanolique d'hexétidine et d'amarante, la température de l'air fluidisé étant maintenue à 40°C ;
- le séchage du mannitol ainsi aspergé dans le lit d'air fluidisé jusqu'à l'obtention d'un taux d'humidité relative inférieure à 2% ;
- le tamisage du granulé résultant sur une grille présentant des mailles de 2 mm de diamètre ;
- le mélange pendant 3 minutes de ce granulé avec 840 g d'aspartam micronisé et 3,360 kg d'un arôme menthe dans un mélangeur souple (vitesse : 30 tours/mn) ; et
- la mise en sachets du mélange résultant

### EXEMPLE 2 : Composition comprenant du digluconate de chlorhexidine et du chlorobutanol hémihydraté

On prépare 100 000 sachets renfermant chacun 590,171 mg d'une composition se présentant sous la forme de granulés et ayant la formulation suivante :
Par sachet
- Digluconate de chlorhexidine à 20% 90,000 mg (soit 18,000 mg en chlorhexidine base)
- Chlorobutanol hémihydraté 90,000 mg
- Colorant Ponceau 4R E124 0,171 mg
- Mannitol granulé 432,000 mg
- Aspartam micronisé 25,000 mg
   Arôme menthe 25,000 mg
par un procédé comprenant :
- la dissolution de 9.45 kg de chlorobutanol hémihydraté dans 4,54 litres d'éthanol ;
- la dissolution de 17,955 g de colorant Ponceau dans 9,45 kg d'une solution aqueuse à 20% de digluconate de chlorhexidine ;
- la mise en fluidisation pendant 15 minutes de 45,36 kg de mannitol granulé dans un lit d'air fluidisé présentant une température de 40°C ;
- l'aspersion du mannitol granulé par la solution éthanolique de chlorobutanol hydraté, puis par la solution de digluconate de chlorhexidine et de colorant Ponceau, la température de l'air fluidisé étant maintenue à 40°C ;
- le séchage du mannitol ainsi aspergé dans le lit d'air fluidisé, jusqu'à l'obtention d'un taux d'humidité relative inférieure à 2% ;
- le tamisage du granulé résultant sur une grille présentant des mailles de 2 mm de diamètre ;
- le mélange pendant 3 minutes de ce granulé avec 2,625 kg d'aspartam micronisé et 2,625 kg d'un arôme menthe dans un mélangeur souple (vitesse : 30 tours/mn) ; et
- la mise en sachets du mélange résultant.

### EXEMPLE 3 : Aptitude des compositions à former des solutions homogènes et stables après dispersion dans l'eau

L'aptitude des compositions préparées conformément aux exemples 1 et 2 - et dénommées ci-après compositions 1 et 2 - à former des solutions homogènes et stables après dispersion dans l'eau a été mise en évidence, d'une part, visuellement pour vérifier l'absence de phénomène de déphasage, et, d'autre part, par des analyses visant à mesurer le diamètre des particules présentes dans des échantillons obtenus par dispersion de ces compositions dans l'eau.

Les échantillons ont été préparés en dispersant le contenu d'un sachet - soit 540,54 mg de composition pour la composition 1 et 590,171 mg de composition pour la composition 2 - dans 20 ml d'eau, et les analyses ont été effectuées au moyen d'un granulomètre à diffraction laser (laser rouge He-Ne de 2 mW minimum, de 18 mm de diamètre et de 633 nm de longueur d'onde) MASTERSIZER BANC LONG (Société MALVERN INSTRUMENTS SA) et d'un échantillonneur MS 1 de la même Société.

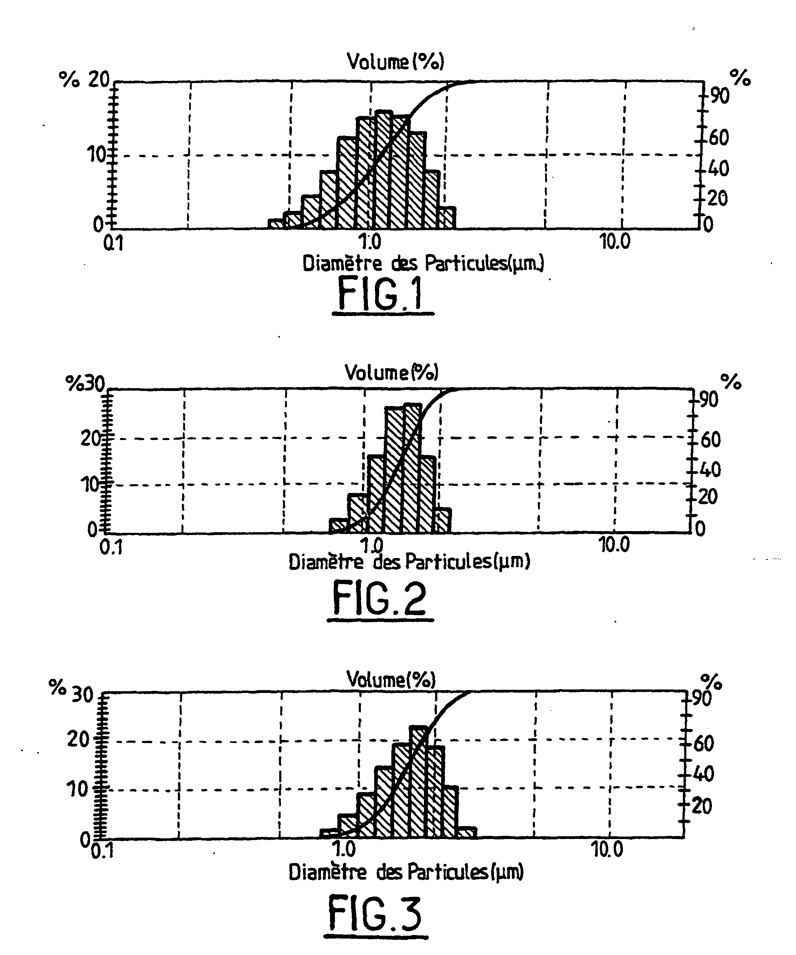

Les Figures 1 à 3 illustrent, à la fois sous la forme d'histogrammes et de courbes, les distributions granulométriques obtenues respectivement pour les échantillons préparés à partir de la composition 1 et de la composition 2, et pour une solution hydro-éthanolique pour bain de bouche de référence : l'HEXTRIL®, qui a l'hexétidine pour principe actif, comme la composition 1.

Sur les Figures 1 à 3, l'axe des abscisses représente le diamètre, exprimé en µm, des particules présentes dans les solutions, l'axe des ordonnées de gauche représente le pourcentage de chaque population de particules - une population correspondant à un rectangle hachuré -, tandis que l'axe des ordonnées de droite représente les pourcentages cumulés des populations de particules.

Ces Figures montrent que les échantillons préparés à des compositions 1 et 2 et la solution d'HEXTRIL® présentent des distributions granulométriques tout à fait comparables et que, dans tous les cas, aucune des particules détectées ne présente une taille supérieure à 2,28 µm. Notamment, la distribution granulométrique de la solution d'HEXTRIL® se caractérise par un pic entre 1,68 et 1,95 µm, assez similaire à celui observé pour les échantillons obtenus à partir de la composition 1, témoignant d'une dissolution de l'hexétidine dans ces échantillons comparable à celle observée dans la spécialité de référence.

## Revendications

1. Procédé de préparation de granulés hydrosolubles renfermant au moins un antiseptique, comprenant :
a) une étape de granulation par voie humide ou d'imprégnation d'un support hydrosoluble pharmaceutiquement acceptable sous forme de granulés au moyen d'un solvant renfermant au moins un principe actif, et
b) une étape de séchage des granulés,
**caractérisé par le fait que** le support hydrosoluble sous forme de granulés est du mannitol et que le principe actif est un antiseptique.

2. Procédé selon la revendication 1, **caractérisé en ce que** le solvant est l'eau, un alcool ou un mélange eau-alcool.

3. Procédé selon la revendication 2, **caractérisé en ce que** l'alcool est l'éthanol.

4. Procédé selon l'une quelconque des revendications précédentes, **caractérisé en ce que** l'antiseptique est choisi parmi les ammonium quaternaires, l'hexétidine, les bisbiguanides, la chlorhexidine et ses sels, le chlorobutanol hémihydraté, l'eugénol, l'eucalyptol, la tyrothricine, et les huiles essentielles.

5. Procédé selon l'une quelconque des revendications précédentes, **caractérisé en ce que** la granulation ou l'imprégnation du mannitol est réalisée avec une quantité de solvant correspondant à 10 à 80% et, de préférence, à 15 à 75% en poids du poids du mannitol.

6. Procédé selon l'une quelconque des revendications précédentes, **caractérisé en ce que** le solvant présente une teneur pondérale en antiseptique comprise entre 10 et 80% et, de préférence, entre 15 et 70%.

7. Procédé selon l'une quelconque des revendications précédentes, **caractérisé en ce que**, à l'issue du séchage des granulés, ces derniers présentent avantageusement un rapport pondéral entre le mannitol et l'antiseptique compris entre 0,5 et 50 et, de préférence, entre 1 et 30.

8. Procédé selon l'une quelconque des revendications précédentes, **caractérisé en ce que** la granulation du mannitol et le séchage des granulés sont réalisés dans un lit d'air fluidisé.

9. Procédé selon l'une quelconque des revendications précédentes,
**caractérisé en ce qu'**il comprend, postérieurement au séchage des granulés, une opération de calibrage de ces granulés.

10. Utilisation d'un procédé selon l'une quelconque des revendications 1 à 9, pour la préparation d'une composition pharmaceutique se présentant sous forme sèche et apte à donner extemporanément une solution pour bains de bouche, après dispersion dans l'eau.

11. Composition pharmaceutique se présentant sous une forme sèche pour réaliser de manière extemporanée une solution pour bains de bouche, par dispersion dans l'eau, **caractérisée en ce qu'**elle comprend des granulés formés de mannitol lié à au moins un antiseptique, lesdits granulés étant préparés par un procédé selon l'une quelconque des revendications 1 à 9.

12. Composition selon la revendication 11, **caractérisée en ce que** l'antiseptique est choisi parmi les ammonium quaternaires, l'hexétidine, les bisbiguanides, la chlorhexidine et ses sels, le chlorobutanol hémihydraté, l'eugénol, l'eucalyptol, la tyrothricine, et les huiles essentielles.

13. Composition selon la revendication 12, **caractérisé en ce que** l'antiseptique est choisi parmi l'hexétidine et le chlorobutanol hémihydraté, éventuellement en association avec du digluconate de chlorhexidine.

14. Composition selon l'une quelconque des revendications 11 à 13, **caractérisée en ce qu'**elle comprend un ou plusieurs édulcorants et/ou un ou plusieurs arômes.

15. Composition selon l'une quelconque des revendications 11 à 14, **caractérisée en ce qu'**elle se présente sous la forme d'une poudre ou de comprimés.

16. Composition selon l'une quelconque des revendications 11 à 15, **caractérisé en ce que** la composition est conditionnée en doses unitaires correspondant chacune à un bain de bouche.

17. Composition selon la revendication 16, **caractérisée en ce que** chaque dose unitaire est conditionnée dans un emballage étanche à l'eau et à l'air, et dont les dimensions sont choisies de manière à pouvoir contenir, une fois ouvert, un volume d'eau suffisant pour effectuer un bain de bouche.

18. Solution pour bains de bouche, **caractérisée en ce qu'**elle est obtenue en dispersant dans l'eau une composition pharmaceutique selon l'une quelconque des revendications 11 à 17.

## Patentansprüche

1. Verfahren zur Herstellung eines wasserlöslichen Granulats, das mindestens ein Antiseptikum enthält, wobei das Verfahren umfaßt:
a) einen Schritt des Granulierens auf feuchtem Wege oder durch Imprägnieren eines pharmazeutisch unbedenklichen wasserlöslichen Trägers in Form eines Granulats mit Hilfe eines Lösungsmittels, das mindestens einen Wirkstoff enthält, und
b) einen Schritt des Trocknens des Granulats,
**dadurch gekennzeichnet, daß** der wasserlösliche Träger in Granulatform Mannitol ist, und daß der Wirkstoff ein Antiseptikum ist.

2. Verfahren nach Anspruch 1, **dadurch gekennzeichnet, daß** das Lösungsmittel Wasser, ein Alkohol oder eine Mischung aus Wasser und Alkohol ist.

3. Verfahren nach Anspruch 2, **dadurch gekennzeichnet, daß** der Alkohol Ethanol ist.

4. Verfahren nach einem der vorhergehenden Ansprüche, **dadurch gekennzeichnet, daß** das Antiseptikum aus quaternären Ammonia, Hexetidin, Bisguaniden, Chlorhexidin und dessen Salzen, Chlorbutanol-Hemihydrat, Eugenol, Eukalyptol, Tyrothricin und ätherischen Ölen ausgewählt ist.

5. Verfahren nach einem der vorhergehenden Ansprüche, **dadurch gekennzeichnet, daß** das Granulieren oder das Imprägnieren des Mannitol mit einer Lösungsmittelmenge vorgenommen wird, die 10 bis 80 Gew.-% und bevorzugt 15 bis 75 Gew.-% des Gewichts des Mannitol entspricht.

6. Verfahren nach einem der vorhergehenden Ansprüche, **dadurch gekennzeichnet, daß** das Lösungsmittel einen Gewichtsanteil an Antiseptikum aufweist, der zwischen 10 und 80% und bevorzugt zwischen 15 und 70% liegt.

7. Verfahren nach einem der vorhergehenden Ansprüche, **dadurch gekennzeichnet, daß** das Granulat nach dem Trocknen vorteilhaft ein Gewichtsverhältnis von Mannitol und dem Antiseptikum von zwischen 0,5 und 50 und bevorzugt von zwischen 1 und 30 aufweist.

8. Verfahren nach einem der vorhergehenden Ansprüche, **dadurch gekennzeichnet, daß** das Granulieren des Mannitol und das Trocknen des Granulats in einer Wirbelluftschicht durchgeführt werden.

9. Verfahren nach einem der vorhergehenden Ansprüche, **dadurch gekennzeichnet, daß** es nach dem Trocknen des Granulats einen Arbeitsschritt des Kalibrierens dieses Granulats umfaßt.

10. Verwendung eines Verfahrens nach einem der Ansprüche 1 bis 9 zur Herstellung einer pharmazeutischen Zusammensetzung, die in trockener Form vorliegt und geeignet ist, nach dem Dispergieren in Wasser an Ort und Stelle eine Lösung für Duschen zu ergeben.

11. Pharmazeutische Zusammensetzung, die in trockener Form vorliegt, zum Bereiten einer Lösung für Duschen an Ort und Stelle durch Dispergieren in Wasser,
**dadurch gekennzeichnet, daß** sie ein Granulat aufweist, das aus Mannitol besteht, welches an mindestens ein Antiseptikum gebunden ist, wobei das Granulat mit einem Verfahren nach einem der Ansprüche 1 bis 9 hergestellt ist.

12. Zusammensetzung nach Anspruch 11, **dadurch gekennzeichnet, daß** das Antiseptikum aus quaternären Ammonia, Hexetidin, Bisguaniden, Chlorhexidin und dessen Salzen, Chlorbutanol-Hemihydrat, Eugenol, Eukalyptol, Tyrothricin und ätherischen Ölen ausgewählt ist.

13. Zusammensetzung nach Anspruch 12, **dadurch gekennzeichnet, daß** dasAntiseptikum aus Hexetidin und Chlorbutanol-Hemihydrat, gegebenenfalls in Verbindung mit Chlorhexidindigluconat, ausgewählt ist.

14. Zusammensetzung nach einem der Ansprüche 11 bis 13, **dadurch gekennzeichnet, daß** sie ein oder mehrere Süßungsmittel und/oder einen oder mehrere Aromastoffe umfaßt.

15. Zusammensetzung nach einem der Ansprüche 11 bis 14, **dadurch gekennzeichnet, daß** sie in Form eines Pulvers oder von Preßtabletten vorliegt.

16. Zusammensetzung nach einem der Ansprüche 11 bis 15, **dadurch gekennzeichnet, daß** die Zusammensetzung in Einheitsdosen abgepackt ist, die jeweils einer Dusche entsprechen.

17. Zusammensetzung nach Anspruch 16, **dadurch gekennzeichnet, daß** jede Einheitsdosis in einer wasser- und luftdichten Verpackung abgepackt ist, deren Abmessungen so gewählt sind, daß sie im geöffneten Zustand ein ausreichendes Wasservolumen zum Vornehmen einer Dusche enthalten kann.

18. Lösung für Duschen, **dadurch gekennzeichnet, daß** sie durch Dispergieren einer pharmazeutischen Zusammensetzung nach einem der Ansprüche 11 bis 17 in Wasser erhalten wird.

## Claims

1. Method of preparing water-soluble granules containing at least one antiseptic, comprising;
a) a step of granulation by a wet method or of impregnation of a pharmaceutically acceptable water-soluble carrier in the form of granules by means of a solvent containing at least one active principle, and
b) a step of drying the granules,
**characterised by** the fact that the water-soluble support in the form of granules is mannitol and the active principle is an antiseptic.

2. Method according to Claim 1, **characterised in that** the solvent is water, an alcohol or a water-alcohol mixture.

3. Method according to Claim 2, **characterised in that** the alcohol is ethanol.

4. Method according to any one of the preceding claims, **characterised in that** the antiseptic is chosen from amongst quaternary ammonia, hexetidine, bisbiguanides, chlorhexidine and salts thereof, chlorobutanol hemihydrate, eugenol, eucalyptol, tyrothricine and essential oils.

5. Method according to any one of the preceding claims, **characterised in that** the granulation or impregnation of mannitol is carried out with a quantity of solvent corresponding to 10% to 80% and preferably 15% to 75% by weight of the weight of mannitol.

6. Method according to any one of the preceding claims, **characterised in that** the solvent has a content by weight of antiseptic of between 10% and 80% and preferably between 15% and 70%.

7. Method according to any one of the preceding claims, **characterised in that**, at the end of the drying of the granules, the latter advantageously have a ratio by weight between the mannitol and antiseptic of between 0.5 and 50 and preferably between 1 and 30.

8. Method according to any one of the preceding claims, **characterised in that** the granulation of the mannitol and the drying of the granules are carried out in a fluidised air bed.

9. Method according to any one of the preceding claims, **characterised in that** it comprises, subsequently to the drying of the granules, an operation of calibrating these granules.

10. Use of a method according to any one of Claims 1 to 9, for the preparation of a pharmaceutical composition in dry form able to give extemporaneously a solution for mouthwashes, after dispersion in water.

11. Pharmaceutical composition in a dry form for extemporaneously producing a solution for mouthwashes, by dispersion in water, **characterised in that** it comprises granules formed from mannitol bound to at least one antiseptic, the said granules being prepared by a method according to any one of Claims 1 to 9.

12. Composition according to Claim 11, **characterised in that** the antiseptic is chosen from amongst quaternary ammonia, hexetidine, bisbiguanides, chlorhexidine and salts thereof, chlorobutanol hemihydrate, eugenol, eucalyptol, tyrothricine and essential oils.

13. Composition according to Claim 12, **characterised in that** the antiseptic is chosen from amongst hexetidine and chlorobutanol hemihydrate, possibly in association with chlorhexidine digluconate.

14. Composition according to any one of Claims 11 to 13, **characterised in that** it comprises one or more sweeteners and/or one or more flavourings.

15. Composition according to any one of Claims 11 to 14, **characterised in that** it is in the form of a powder or tablets.

16. Composition according to any one of Claims 11 to 15, **characterised in that** the composition is packaged in unitary doses each corresponding to one mouthwash.

17. Composition according to Claim 16, **characterised in that** each unitary dose is packaged in a watertight and airtight package whose dimensions are chosen so as to be able contain, once opened, a volume of water sufficient to effect a mouthwash.

18. Solution for mouthwashes, **characterised in that** it is obtained by dispersing in water a pharmaceutical composition according to any one of Claims 11 to 17.
